# EUROPEAN PATENT SPECIFICATION

(11) **EP 2 522 426 B1**
(45) Date of publication and mention of the grant of the patent: **12.02.2014**
(21) Application number: 11165779.7
(22) Date of filing: 12.05.2011
(51) Int. Cl.: B01J 35/00, C07C 51/265

(54) **Method for preparing carboxylic acids**
Verfahren zur Herstellung von Carboxylsäuren
Méthode pour la production d'acides carboxyliques

(43) Date of publication of application: 14.11.2012
(73) Proprietor: King Saud University, 11421 Riyadh (SA)
(72) Inventor: Al-Zahrani, Saeed Mohammed, 11421 Riyadh (SA); Richeni, Horacio Falcon, 28049 Madrid (ES); Salvador, Pedro Tartaj, 28049 Madrid (ES); Campos Martín, Jose Miguel, 28049 Madrid (ES); Fierro, José Luis García, 28049 Madrid (ES)
(74) Representative: Goddar, Heinz J.

(56) References cited:
- WO-A1-03/090927
- DE-A1- 4 339 139
- US-A1- 2004 024 248
- TAEGHWAN HYEON: "Chemical synthesis of magnetic nanoparticles", CHEMICAL COMMUNICATIONS, no. 8, 3 April 2003 (2003-04-03), pages 927-934, XP55003772, ISSN: 1359-7345, DOI: 10.1039/b207789b
- ANDRÉS GUERRERO-MARTÍNEZ ET AL: "Recent Progress on Silica Coating of Nanoparticles and Related Nanomaterials", ADVANCED MATERIALS, vol. 22, no. 11, 19 March 2010 (2010-03-19), pages 1182-1195, XP55003773, ISSN: 0935-9648, DOI: 10.1002/adma.200901263

## Description

The present invention relates to a method for preparing carboxylic acids utilizing a heterogeneous catalyst.

Carboxylic acids are important compounds in the field of chemistry, both for direct industrial use or as intermediate product for further conversion. For example, carboxylic acids derived from alkyl aromatics are a starting material to produce polyester fiber, polyester film, bottles, etc. The polyester fibers are used in textile goods and for industrial use as well, such as tire code, and the polyester film being coated with adhesive or emulsion is useful for wrapping tape, photographic film, and recording tape. One very important material in this regard is terephthalic acid, which is a staring material to prepare polyethylene terephthalate which is a major polymer of polyester fiber, film, bottles, etc.

In a conventional method for preparing carboxylic acids, such as terephthalic acid, it is well known that para-xylene is oxidized by an oxygen molecule in acetic acid solvent in the presence of a catalytic system. Suitable oxidation catalyst compositions include a cobalt compound and a manganese compound, usually in combination with a promoter, such as a bromine compound. See, for example, U.S. 2,833,816, 2,962,361, 3,089,906, 3,970,696, 4,159,307, 4,314,073 4,327,226, 5,679,847, 5,756,833, U.S. 2002/0193630 A1, U.S. 2002/0183546, JP 1997278709 A, GB 1 389 478 and WO2006/096311.

The process conditions in the prior art are highly corrosive due to incorporation of acetic acid and bromine to the reaction medium and therefore reactor and process equipment must be built up with titanium and titanium alloys instead of stainless steel, see, U.S. Pat. No. 3,012,038.

The resulting crude carboxylic acid, such as terephthalic acid, is only slightly soluble in the acetic acid solvent under the reaction conditions and precipitates from the solvent to form a suspension that includes terephthalic acid solid, a solvent acting as the suspending medium for the solids and containing a small amount of dissolved terephthalic acid, catalyst components, unreacted p-xylene, incompletely oxidized intermediate oxidation products such as para-tolualdehyde, para-toluic acid, and 4-carboxybenzaldehyde; and organic impurities that are known to cause discoloration, and especially fluorenones. The crude terephthalic acid composition is discharged from the oxidation zone and subjected to any of several mother liquor exchange, separation, purification, or recovery methods, with the recovered solvent and catalyst composition being recycled either directly back to the oxidation reaction or after processing, such as by catalyst recovery or solvent purification.

One key challenge for the commercial development and practical use of homogeneous catalysts is the separation of product from the catalytic media. This process is often complicated and usually accomplished by means of complex work-up procedures. Attempts to improve catalyst recovery and recycling include the use of biphasic system or the immobilisation of catalysts on solids. Anchoring metal complexes often require chemical modifications on the support and ligands, which can influence on the catalytic performance. For these systems, the most commonly used supports are silica, alumina, and carbon, and the product separation is frequently achieved by a physical method such as filtration or even centrifugation. However, as the size of the support decreases, the separation becomes a difficult task. For systems comprised of very thin solids, simple filtration constitutes an inefficient tool to accomplish product isolation.

DE 43 39 139 A1 relates to a catalyst comprising a magnetizable core, which is, if necessary, coated by a binding agent and the surface of which carries a catalytic active metal or metal compound.

Thus, there is a need in industry for a method for preparing carboxylic acids by liquid phase oxidation with an oxygen containing gas in the presence of a catalyst which method allows easy separation and recycling steps of the catalyst.

The object of the present invention is therefore to provide a heterogeneous catalyst which overcomes the difficulties and drawbacks of the prior art, especially which allows easy separation and recycling. Further, a method for preparing carboxylic acid shall be provided.

The object is achieved by a method for preparing carboxylic acids according to claim 1 of the present application. Preferred embodiments of the inventive method result from claims 2 to 12. Described herein is a heterogeneous catalyst for preparing carboxylic acids, the catalyst comprising nanoparticles which comprise a support containing a magnetic core and a shell of an inorganic oxide layer, wherein at least one organic or inorganic metal salt and an imide compound are deposited in and/or on the inorganic oxide layer.

According to the invention is a method for preparing carboxylic acids comprising the step of contacting an aromatic hydrocarbon comprising at least one group including an α C-atom that is oxidizable into a carboxylic group in liquid phase with an oxygen containing gas in the presence of a heterogeneous catalyst comprising nanoparticles which comprise a support containing a magnetic core and a shell of an inorganic oxide layer, wherein at least one organic or inorganic metal salt and an imide compound are deposited in and/or on the inorganic oxide layer.

It is a core feature of the present invention that the heterogeneous catalyst is based on nanoparticles as support that contain a magnetic core coated by a shell of an inorganic oxide layer. The inorganic oxide layer has to be porous having a high specific surface. With such a support structure, the heterogeneous catalyst of the method of the present invention can be easily and fast removed and recovered from the reaction medium. The heterogeneous catalyst can be attracted into relatively low static magnetic field strengths and can be easily recovered since no residual magnetization is observed when the magnetic field is removed. The use of magnetic nanoparticle coated by a porous inorganic oxide shell for the immobilization of the active catalyst components (metal salt and imide) have been undertaken to obtain the nanoparticle-type catalyst, with enlarged stability in the liquid phase oxidation of the starting compounds.

The magnetic nanoparticles can be prepared by methods known in the art, for example T. Hyeon Chem. Commun., 2003, 927-934, or R. D. Rieke et al., Chem. Mater., 1996, 8, 1770.

The particle size of the magnetic nanoparticles is in the order of nanometers, 1-1000 nanometer, more preferably between 10-100 nanometer.

Preferably, the magnetic core comprises hematite.

The inorganic oxide layer can be incorporated by any technique well-known in the state of the art, see for example A. Guerrero-Martínez, et al., Adv. Mater. 2010, 22, 1182-1195.

More preferably, the inorganic oxide layer comprises at least one oxide of the elements of groups 2 to 14 of the periodic table of elements, preferably SiO₂, Al₂O₃, zeolites, B₂O₃, GeO₂, Ga₂O₃, ZrO₂, TiO₂, MgO or mixtures thereof, most preferably silica or its mixtures with another inorganic oxide.

The inorganic oxide layer should be thin enough to avoid blocking of the magnetic properties of the magnetic nanoparticles, but thick enough to protect it against the reaction environment. The size of the inorganic oxide layer is preferably between 1-100 nanometer, more preferably between 5-20 nanometer.

In one embodiment, the metal of the inorganic or organic metal salt is selected from the group consisting of cobalt, magnesium, chromium, copper, nickel, vanadium, iron, molybdenum, tin, cerium, zirconium and mixtures thereof

As already mentioned above, the metal salts that have a variable valency can be used in the form of an inorganic or organic salt, preferably an organic salt, more preferably a lower carboxylic acid salt is used, like metal acetate. Preferably, a cobalt salt is used as catalyst, more preferably in combination with a manganese, cerium and/or zirconium salt. A catalyst comprising a combination of cobalt and manganese salt is most preferred, in view of its good activity and stability.

The ratio of cobalt to manganese may vary widely, for example from 5:1 to 1:25, but is preferably from 2:1 to 1:15, more preferably from 1 to 1:10 or even about 1:5.

Even preferred, the amount of the heterogeneous catalyst ranges from 0.0001 to 1 wt %, preferably from 0.001 to 0.5 wt %, based on the amount of liquid in the reactor.

The amount of catalyst that is used in the method according to the present invention may vary widely, for example from several ppm to some percentage. In case cobalt and manganese are used as catalyst, their concentrations are preferably in the range of 10 to 10.000 ppm (1%) and 20 to 20.000 ppm (2%), respectively, based on mass of solvent. More preferably cobalt and manganese concentrations are in the range of 100 to 1.000 ppm and 500 to 2.000 ppm, respectively.

Preferably, the support, constituted by the inorganic oxide layer on the magnetic core, has a specific surface area according to BET method of between 20 and 1500 m²/g, preferably 100-1000 m²/g, and/or a pore volume of between 0.1 and 3 ml/g.

The amount of the imide compound is, for example, preferably from 0.000001 to about 0.1 mole per mol of aromatic hydrocarbon.

Suitable alkyl substituted aromatic feed materials for the oxidation generally comprise an aromatic hydrocarbon substituted at one or more positions, normally corresponding to positions of the carboxylic acid groups of the aromatic carboxylic acid being prepared, with at least one group that includes an α C-atom that is oxidizable to a carboxylic acid group. The oxidizable substituent or substituents can be alkyl groups, such as a methyl, ethyl or isopropyl, or groups already containing oxygen, such as formyl, acyl or hydroxyalkyl groups. Substituents can be the same or different. The aromatic ring can be a benzene nucleus or bi- or polycyclic, such as a naphthalene nucleus. The number of oxidizable substituents of the aromatic compound can equal the number of sites available on the aromatic ring, but is generally less, preferably 1 or 2, and most preferably 2. Examples of useful feed compounds, which can be used alone or in combinations, include toluene, ethylbenzene and other alkyl-substituted benzenes, o-xylene, p-xylene, m-xylene, tolualdehydes, toluic acids, alkyl benzyl alcohols, 1-formyl-4-methylbenzene, 1-hydroxymethyl-4-methylbenzene, methylacetophenone, 1,2,4-trimethylbenzene, 1-formyl-2,4-dimethylbenzene, 1,2,4,5-tetramethyl-benzene, alkyl-, formyl-, acyl-, and hydroxylmethylsubstituted naphthalenes, such as 2,6-dimethylnaphthalene, 2,6-diethylnaphthalene, 2,7-dimethylnaphthalene, 2,7-diethylnaphthalene, 2-formyl-6-methylnaphthalene, 2-acyl-6-methyl-naphthalene, 2-methyl-6-ethylnaphthalene and partially oxidized derivatives thereof.

Any oxygen containing gas can be applied, like molecular oxygen, air or any other gas mixture comprising oxygen, e.g. carbon dioxide. In a preferred way of performing the method according to the invention the oxygen containing gas comprises 4-50 volume percent of carbon dioxide, preferably 10-25 volume percent. This further reduces reaction time and side-reactions. The ratio of total amount of oxygen to the substituted aromatic compound is depending on the number of substituents to be oxidized. Preferably, oxygen is used in excess; for example the molar ratio of oxygen to aromatic compound is from 3 to 500, more preferably from 5 to 100. The method of the present invention is conducted in a liquid phase. The method can be conducted in the presence or absence of solvents.

When a solvent is used, water, (mono)carboxylic acid and their mixtures are preferred, but other suitable solvents or liquid media can be used. Preferred solvents for aromatic feed materials in the liquid phase reaction comprise low molecular weight monocarboxylic acids and preferably a C₁-C₁₄ monocarboxylic acid, for example acetic acid, propionic acid, butyric acid, valeric acid and benzoic acid. Acetic acid is a preferred monocarboxylic acid. The amount of solvent that is used is not critical, but preferably the ratio of solvent to aromatic compound is in the range of 3:1 1 to 15:1, if present at all.

Even preferred, a free-radical generator and/or free radical reaction accelerator is added, such as halogen, peracid, peroxide, nitric acid, nitrous acid or salts thereof.

As free-radical generator or free-radical reaction accelerator, components may be chosen, which are not limited to halogens (such as chlorine and bromine), peracids (such as peracetic acid and m-chloroperbenzoic acid), and peroxides (such as hydrogen peroxide, t-butyl hydroperoxide, and other hydroperoxides), as well as nitric acid, nitrous acid, and salts thereof. The presence of these component(s) may enhance the reaction. The amount of these component(s) is preferably from 0.001 to 20 mole per mole of the imide compound.

Finally, it is preferred that the method is carried out at a temperature in the range of 80-200°C, preferably 100-175°C, and/or a pressure at a range of 1.0-2.5 MPa, and/or the method is carried out in a reactor having a residence time of 60 to 120 minutes.

The reaction zone in the method according to the present invention can include one or more reactors as are known to skilled persons, for example a stirred tank reactor that may be operated in a continuous or batch-wise way.

The method of the present invention may further comprise additional steps to isolate and purify the carboxylic acid as obtained by the method as described above. Such processing steps are well known to the skilled person, and have been described in general literature.

As already stated above, active catalyst supported is composed of at least one metal salt and an imide compound. These compounds are deposited on and/or in the surface of the magnetic support, i.e. the oxide layer. The incorporation of the catalytic components to the magnetic support nanoparticles can be made by any person skilled in the art, for example by wet impregnation, dry impregnation, covalent bonding, tethering, etc.

The magnetic field gradient to be applied may vary in a broad range and can be established by known methods, for example a permanent magnet or a wire conducting current. To "apply" a magnetic field means to influence the obtained system of continuous phase, dispersed phase and nanoparticles by magnetic field.

The magnetic field applied may be a constant field or a time dependent field, as the specific method requires.

Additional features and advantageous of the inventive catalyst and method shall be further illustrated on the basis of the following examples.

### Examples

### Example 1: Coated nanoparticle synthesis

Firstly, Na₂CO₃ 0.45 M and FeSO₄ 0.075 M solutions were stirred at 40°C under airflow. The formed precipitate was centrifuged and dried. The solid obtained, was dispersed in water and HNO₃ with a concentration 6 g/L and pH = 4, then the solution was centrifuged and the solid was dried.

Secondly, the solid obtained above was dispersed in a negatively charged polyelectrolyte (poly(styrenesulfonic acid-co-maleic acid) sodium salt,) aqueous solution with a concentration 2 g/L, then ZrOCl₂ were added to reach pH = 3, the solution was centrifuged and the solid was dried.

Thirdly, the solid obtained, was dispersed in a aqueous solution of PDDA (poly(diallyldimethylammonium chloride)) at pH = 12, followed by addition of sodium silicate (2 g/L), the solid obtained was calcined at 300°C, obtaining hematite coated with ZrO₂ and SiO₂.

Fourthly, these nanoparticles were dispersed in a solution tetraethyl orthosilicate and octade-cyltrimethoxysilane with a molar ratio = 5 in a mixtures of ethanol, water and ammonia, after that the solution was centrifuged and the solid was calcined at 400°C.

Finally, these solid was reduced at 400°C under hydrogen flow. This solid have the following textural properties: BET surface area: 309 m²/g, pore volume: 0.44 mL/g and mean pore diameter: 5.6 nm.

### Example 2: Incorporation of active phases

5 g of silica coated magnetic nanoparticles powder prepared in Example 1 was added to 200 mL of acetonitrile solution of N-hydroxyphthalimide (20 g/L), cobalt (II) acetate (0.76 g/L) and manganese (II) acetate (0.53 g/L). The mixture was refluxed for 12 h to disperse the compounds throughout the support. Then the solvent was evaporated (at 130 mbar and 60°C), and the powder was dried for an additional 12 h at 70°C.

### Comparative Example 1

Following the procedure of US Pat. Publn. No. US 2004/0024248. In a 100 mL stainless steel stirred reactor 1.46 g of p-xylene; 21 g of acetic acid, 0.0166 g of cobalt(II) acetate, 0.0115 g of manganese(II) acetate and 0.435 g of N-hydroxyphthalimide were mixed. The reactor was heated to 90°C, and the pressure was increased to 30 bar with air. The stirring was started up (1500 rpm) to initiate the reaction. After a reaction time of 3 h, the p-xylene conversion was 100 % and the selectivity to terephthalic acid was 62 % and to p-toluic acid 29 %.

### Example 3:

In a 100 mL stainless steel stirred reactor 1.46 g of p-xylene; 21 g of acetic acid and 0.833 g of solid prepared in Example 2 were mixed. The reactor was heated to 90°C, and the pressure was increased to 30 bar with air. The stirring was started up (1500 rpm) to initiate the reaction. After a reaction time of 3 h, the p-xylene conversion was 100 % and the selectivity to terephthalic acid was 58 % and to p-toluic acid 41 %. After 5 h of reaction the selectivity to terephthalic acid was 66 % and to p-toluic acid 33 %. When the reaction has finished the catalysts were recovered with a commercial magnet (NdFeB magnet with a magnetic field B of 0.7 Tesla).

### Comparative Example 2:

Following the procedure of US Pat. Publn. No. US 2004/0024248. In a 100 mL stainless steel stirred reactor 1.46 g of p-xylene; 21 g of acetic acid, 0.0166 g of cobalt(II) acetate, 0.0115 g of manganese(II) acetate and 0.435 g of N-hydroxyphthalimide were mixed. The reactor was heated to 90°C, and the pressure was increased to 50 bar with air. The stirring was started up (1500 rpm) to initiate the reaction. After a reaction time of 3 h, the p-xylene conversion was 100% and the selectivity to terephthalic acid was 67 % and to p-toluic acid 32 %.

### Example 4:

In a 100 mL stainless steel stirred reactor 1.46 g of p-xylene; 21 g of acetic acid and 0.833 g of solid prepared in Example 2 were mixed. The reactor was heated to 90°C, and the pressure was increased to 50 bar with air. The stirring was started up (1500 rpm) to initiate the reaction. After a reaction time of 3 h, the p-xylene conversion was 100 % and the selectivity to terephthalic acid was 66 % and to p-toluic acid 33 %. When the reaction has finished the catalysts were recovered with a commercial magnet (NdFeB magnet with a magnetic field B of 0.7 Tesla).

### Comparative Example 3:

Following the procedure of US Pat. Publn. No. US 2004/0024248. In a 100 mL stainless steel stirred reactor 1.46 g of p-xylene; 21 g of acetic acid, 0.0166 g of cobalt(II) acetate, 0.0115 g of manganese(II) acetate and 0.435 g of N-hydroxyphthalimide were mixed. The reactor was heated to 90°C, and the pressure was increased to 10 bar with oxygen. The stirring was started up (1500 rpm) to initiate the reaction. After a reaction time of 3 h, the p-xylene conversion was 100 % and the selectivity to terephthalic acid was 79 % and to p-toluic acid 18 %.

### Example 5:

In a 100 mL stainless steel stirred reactor 1.46 g of p-xylene; 21 g of acetic acid and 0.833 g of solid prepared in Example 2 were mixed. The reactor was heated to 90°C, and the pressure was increased to 10 bar with oxygen. The stirring was started up (1500 rpm) to initiate the reaction. After a reaction time of 3 h, the p-xylene conversion was 100 % and the selectivity to terephthalic acid was 75 % and to p-toluic acid 23 %. When the reaction has finished the catalysts were recovered with a commercial magnet (NdFeB magnet with a magnetic field B of 0.7 Tesla).

The features disclosed in the foregoing description and the claims may, both separately and in any combination thereof, be material for realising the invention in diverse forms thereof.

## Claims

1. Method for preparing carboxylic acids comprising the step of contacting an aromatic hydrocarbon comprising at least one group including an a C-atom that is oxidizable into a carboxylic group in liquid phase with an oxygen containing gas in the presence of a heterogeneous catalyst comprising nanoparticles which comprise a support containing a magnetic core and a shell of an inorganic oxide layer, wherein at least one organic or inorganic metal salt and an imide compound are deposited in and/or on the inorganic oxide layer.

2. Method according to claim 1, wherein the magnetic core comprises hematite.

3. Method according to claim 1 or 2, wherein the inorganic oxide layer comprises at least one oxide of the elements of groups 2 to 14 of the periodic table of elements, preferably SiO₂, Al₂O₃, zeolites, B₂O₃, GeO₂, Ga₂O₃, ZrO₂, TiO₂, MgO or mixtures thereof, most preferably silica or its mixtures with another inorganic oxide.

4. Method according to any of the preceding claims, wherein the metal of the inorganic or organic metal salt is selected from the group consisting of cobalt, magnesium, chromium, copper, nickel, vanadium, iron, molybdenum, tin, cerium, zirconium and mixtures thereof.

5. Method according to any of the preceding claims, wherein the support, constituted by the inorganic oxide layer on the magnetic core, has a specific surface area according to BET method of between 20 and 1500 m²/g, more preferably between 100 and 1000 m²/g, and/or a pore volume of between 0.1 and 3 ml/g.

6. Method according to any of the preceding claims, wherein the aromatic hydrocarbon is an alkyl substituted hydrocarbon, preferably selected from the group consisting of toluene, ethyl benzene, p-xylene, o-xylene and m-xylene.

7. Method according to any of the preceding claims, wherein the oxygen containing gas is selected from the group consisting of molecular oxygen, air, carbon dioxide or mixture thereof.

8. Method according to any of the preceding claims, wherein the method is carried out in the presence of a solvent, the solvent being preferably selected from the group consisting of water, carboxylic acid and mixtures thereof.

9. Method according to any of the preceding claims, wherein the amount of the heterogeneous catalyst ranges from 0.0001 to 20 wt %, preferably from 0.01 to 10 wt %, based on the amount of liquid in the reactor.

10. Method according to any of the preceding claims, wherein a free-radical generator and/or free radical reaction accelerator is added, such as halogen, peracid, peroxide, nitric acid, nitrous acid or salts thereof.

11. Method according to claim 10, wherein the free-radical generator and/or free-radical reaction accelerator is added in an amount of 0.001 to 20 mole per mole of the imide compound.

12. Method according to any of the preceding claims, wherein the method is carried out at a temperature in the range of 80-200°C, preferably 100-175°C, and/or a pressure at a range of 1.0-2.5 MPa, and/or the method is carried out in a reactor having a residence time of 60 to 120 minutes.

## Patentansprüche

1. Verfahren zum Herstellen von Carbonsäuren umfassend einen Schritt eines Kontaktierens eines aromatischen Kohlenwasserstoffs, umfassend mindestens eine Gruppe, welche ein α-C-Atom einschließt welches in flüssiger Phase mit einem Sauerstoff enthaltenden Gas in eine Carboxylgruppe in der Gegenwart eines heterogenen Katalysators oxidierbar ist, welcher Nanopartikel umfasst, welche einen Trager, enthaltend einen magnetischen Kern und eine Schale aus einer anorganischen Oxidschicht, umfassen, wobei mindestens ein organisches oder anorganisches Metallsalz und eine Imidverbindung in und/oder auf der anorganischen Oxidschicht angelagert sind

2. Verfahren nach Anspruch 1, wobei dei magnetische Kern Hematit umfasst

3. Verfaluen nach Anspruch 1 oder 2, wobei die anorganische Oxidschicht mindestens ein Oxid der Elemente der Gruppen 2 bis 14 des Periodensystems der Elemente, bevorzugt SiO₂, Al₂O₃, Zeolithe, B₂O₃, GeO₂, Ga₂O₃, ZrO₂, TiO₂, MgO oder Gemische hiervon, noch bevorzugter Silika oder dessen Gemische mit einem anderen anorganischen Oxid, umfasst

4. Verfahren nach einem der vorangehenden Anspruche, wobei das Metall des anorganischen oder organischen Metallsalzes ausgewählt ist aus der Gruppe bestehend aus Cobalt, Magnesium, Chrom, Kupfer, Nickel, Vanadium, Eisen, Molybdan, Zinn, Cer, Zirkonium und Gemischen hiervon.

5. Verfahren nach einem der vorangehenden Anspruche, wobei der Trager, welcher durch die anorganische Oxidschicht auf dem magnetischen Kern gebildet wird, einen spezifischen Oberflächenbereich nach dem BET-Verfahren zwischen 20 und 1500 m²/g, noch bevorzugter zwischen 100 und 1000 m²/g und/oder ein Porenvolumen zwischen 0,1 und 3 ml/g aufweist

6. Verfahren nach einem der vorangehenden Ansprüche, wobei der aromatische Kohlenwasserstoff ein Alkyl-substituierter Kohlenwasserstoff, bevorzugt ausgewählt aus der Gruppe bestehend aus Toluol, Ethylbenzen, p-Xylen, o-Xylen und m-Xylen, ist.

7. Verfahren nach einem der vorangehenden Ansprüche, wobei das Sauerstoff enthaltende Gas ausgewählt ist aus der Gruppe, bestehend aus molekularem Sauerstoff, Luft, Kohlendioxid oder Gemischen hiervon

8. Verfahren nach einem der vorangehenden Anspruche, wobei das Verfahren in der Gegenwart eines Losungsmittels durchgeführt wird, wobei das Lösungsmittel bevorzugt ausgewählt ist aus der Gruppe, bestehend aus Wasser, Carbonsaure und Gemischen hiervon.

9. Verfahren nach einem der vorangehenden Ansprüche, wobei die Menge des heterogenen Katalysators von 0,001 bis 20 Gew.%, bevorzugt von 0,01 bis 10 Gew % reicht, basierend auf der Menge der Flüssigkeit in dem Reaktor.

10. Verfahren nach einem der vorangehenden Anspruche, wobei ein freier Radikal-Generator und/oder ein freier Radikal-Reaktionsbeschleuniger, wie Halogen, Persaure, Peroxid, Salpetersäure, salpetrige Saure und Salze hiervon, zugegeben wird.

11. Verfahren nach Anspruch 10, wobei der freie Radikal-Generator und/oder freie Radikal-Reaktionsbeschleuniger in einer Menge von 0,001 bis 20 Mol pro Mol der Imidverbindung zugegeben wird.

12. Verfahren nach einem der vorangehenden Ansprüche, wobei das Verfahren bei einer Temperatur in dem Bereich von 80-200°C, bevorzugt 100-175°C, und/oder einem Druck in einem Bereich von 1,0-2,5 MPa durchgeführt wird, und/oder das Verfahren in einem Reaktor mit einer Verweilzeit von 60 bis 120 Minuten durchgeführt wird.

## Revendications

1. Procédé de préparation d'acides carboxyliques, comprenant l'étape de mise en contact d'un hydrocarbure aromatique comprenant au moins un groupe comprenant un atome de carbone alpha- qui est oxydable en un groupe carboxylique en phase liquide avec un gaz contenant de l'oxygène en présence d'un catalyseur hétérogène comprenant des nanoparticules qui comprennent un support contenant un noyau magnétique et une enveloppe d'une couche d'oxyde inorganique, dans lequel au moins un sel métallique organique ou inorganique et d'un composé imide sont déposés dans et / ou sur la couche d'oxyde inorganique.

2. Procédé selon la revendication 1, dans lequel le noyau magnétique comprend l'hématite.

3. Procédé selon la revendication 1 ou 2, dans lequel la couche d'oxyde inorganique comprend au moins un oxyde d'éléments des groupes 2 à 14 du tableau périodique des éléments, de préférence SiO₂ Al₂O₃, les zéolithes, B₂O₃, GeO₂, Ga₂O₃, ZrO₂, TiO₂, MgO ou des mélanges de ceux-ci, de préférence de la silice ou ses mélanges avec un autre oxyde inorganique.

4. Procédé selon l'une quelconque des revendications précédentes, dans lequel le métal du sel métallique inorganique ou organique est choisi dans le groupe constitué par le cobalt, le magnésium, le chrome, le cuivre, le nickel, le vanadium, le fer, le molybdène, l'étain, le cérium, le zirconium et des mélanges de ceux-ci.

5. Procédé selon l'une quelconque des revendications précédentes, dans lequel le support, constitué par la couche d'oxyde inorganique sur le noyau magnétique, possède une surface spécifique déterminée par la méthode BET comprise entre 20 et 1500 m²/g, plus préférentiellement comprise entre 100 et 1000 m²/g, et/ou un volume poreux compris entre 0,1 et 3 ml/g.

6. Procédé selon l'une quelconque des revendications précédentes, dans lequel l'hydrocarbure aromatique est un hydrocarbure substitué par un groupe alkyle, de préférence choisi dans le groupe constitué par le toluène, l'éthylbenzène, le p-xylène, o-xylène et du m-xylène.

7. Procédé selon l'une quelconque des revendications précédentes, dans lequel le gaz contenant de l'oxygène est choisi dans le groupe constitué par l'oxygène moléculaire, l'air, le dioxyde de carbone ou un mélange de ceux-ci.

8. Procédé selon l'une quelconque des revendications précédentes, dans lequel le procédé est mis en oeuvre en présence d'un solvant, le solvant étant de préférence choisi dans le groupe constitué par l'eau, l'acide carboxylique et leurs mélanges.

9. Procédé selon l'une quelconque des revendications précédentes, dans lequel la quantité de catalyseurs hétérogènes est comprise entre 0,0001 à 20% en poids, de préférence entre 0,01 à 10% en poids, exprimés sur la base de la quantité de liquide dans le réacteur.

10. Procédé selon l'une quelconque des revendications précédentes, dans lequel un générateur de radicaux libres et/ou un accélérateur de réaction à radicaux libres est ajouté, tel qu'un halogène, un peracide, un peroxyde, l'acide nitrique, l'acide nitreux ou des sels en dérivant.

11. Procédé selon la revendication 10, dans lequel un générateur de radicaux libres et/ou un accélérateur de réaction de radicaux libres est ajouté en une quantité de 0,001 à 20 moles par mole du composé imide.

12. Procédé selon l'une quelconque des revendications précédentes, dans lequel le procédé est mis en oeuvre à une température comprise entre 80 et 200°C, de préférence de 100-175°C, et/ou sous une pression comprise entre 1,0 et 2,5 MPa, et/ou le procédé est mis en oeuvre dans un réacteur pendant un temps de séjour de 60 à 120 minutes.
